# EUROPEAN PATENT APPLICATION

(11) **EP 4 101 439 A1**
(43) Date of publication of application: **14.12.2022**
(21) Application number: 22176731.2
(22) Date of filing: 01.06.2022
(51) Int. Cl.: A61K 9/02, A61K 9/14, A61K 9/20, A61K 9/24

(54) **COMPOSITION OF N-PALMITOYL-ETHANOLAMIDE AND BAICALEIN IN CO-MICRONIZED FORM**

(30) Priority: 11.06.2021 IT 202100015278
(71) Applicant: EPITECH GROUP S.p.A., 20144 Milano (MI) (IT)
(72) Inventor: DELLA VALLE, Francesco, deceased (IT); DELLA VALLE, Maria Federica, I-20144 Milano (IT); GOMIERO, Chiara, I-20144 Milano (IT); MARCOLONGO, Gabriele, I-20144 MIilano (IT); CUZZOCREA, Salvatore, I-20144 Milano (IT)
(74) Representative: Long, Giorgio

(57) **Abstract**

It is the object of the present invention a composition based on N-palmitoyl-ethanolamide and Baicalein in the co-micronized form.

In particular, the present invention relates to a composition comprising a mixture of palmitoyl-ethanolamide (PEA) and Baicalein in the co-micronized form.

The composition of the invention is usable for treating benign prostatic hyperplasia.

## Description

### Technical field of the invention

It is the object of the present invention a composition based on N-palmitoyl-ethanolamide and Baicalein in the co-micronized form.

### Background art

Benign prostatic hyperplasia (BPH), also known as benign prostatic hypertrophy, is a benign enlargement of the prostate gland following abnormal cell growth. It is a highly prevalent clinical condition in the male population with a strongly negative impact on patients' quality of life due to gradually occurring obstructive and/or irritative symptoms (pollakiuria and nocturia, intermittent urination, decreased urinary jet strength, strangury and the feeling of incomplete bladder emptying).

The number of health services related to this disease is very high and BPH is the second disease diagnosed every year in the male population in Italy.

The probability of developing BPH after age 50 is about 50% and the percentage rises above 80% in those over 80 years of age.

The etiology is probably multifactorial, due to hormonal changes related to age and inflammatory and metabolic processes. The accumulation of dihydrotestosterone (DHT) and the increase in prostate estrogen levels with advancing age are the main cause of cellular hyperplasia and the increase in prostate size.

The diagnosis is carried out through rectal examination, quantification of irritative and/or obstructive symptoms with a specific questionnaire known as the International Prostate Symptom Score (IPSS), abdominal ultrasound and urinary flow measurement. Although BPH does not degenerate into prostate cancer, these problems are very often diagnosed together: it is thus customary to associate the specific tests for BPH with the dosage of prostate specific antigen (PSA) and/or a biopsy sample for the diagnosis of prostate cancer.

To date, the most common therapy for the treatment of BPH involves the use of drugs which inhibit the enzyme 5α-reductase (5α-red) responsible for the conversion of testosterone, produced by the testes and poured into the prostate, to DHT, with a consequent reduction in the binding of the latter to the adrenergic α-1 receptors (AR) present on the prostate and bladder neck. Thereby there is a greater preservation of the morphology, differentiation, proliferation and survival of the prostate gland cells.

The 5α-red enzyme inhibitors on the market are Finasteride and Dutasteride, which are involved in the inhibition of the conversion of testosterone to DHT responsible for the enlargement of the prostate gland. These drugs are associated with RA antagonists (Alfuzosin, Doxazosin, Tamsulosin, Terazosin), responsible for the muscle relaxation of the prostate and bladder neck, so as to improve the flow of urine and the emptying of the bladder. In parallel to these drugs, phytotherapeutic products, natural products obtained from plants frequently used in traditional Chinese medicine, can also be prescribed.

Unlike what occurs for some types of tumors, there is no specific chemo-prevention for BPH. Although the 5α-red inhibitor drugs and RAs used for BPH are widely used to slow the progression of the disease, they do not fall into the category of chemo-preventive drugs due to the numerous side effects they present (dizziness, erectile dysfunction and cardiovascular risks). Furthermore, the administration of the drug finasteride is responsible for increasing the likelihood of more aggressive prostate tumors with respect to the average and delaying the diagnosis of the tumor with heavy repercussions on the final outcome.

It is thus of fundamental importance to identify a safe and non-toxic treatment capable of slowing the progression of BPH and able to not "mask" the possible presence of a tumor in the prostate gland. In this regard, ultra-micronized Palmitoylethanolamide (um-PEA) has aroused particular interest in recent years for the anti-neuroinflammatory properties thereof, also in BPH.

Baicalein is a flavonoid extracted from the root of *Scutellaria baicalensis* with high antioxidant and antitumor properties. Both substances have demonstrated a very high degree of safety.

### Summary of the invention

The present invention originates from the surprising discovery that, contrary to expectations, the combination of palmitoylethanolamide and Baicalein, when such substances are in the co-micronized form, acts on the apoptotic pathway, significantly decreasing the expression of the anti-apoptotic factor Bcl-2 and significantly increasing the expression of the pro-apoptotic factor Bax in prostate tissue.

An object of the present invention is a composition containing a mixture of palmitoyl-ethanolamide and Baicalein in the co-micronized form (co-micronized PEA-Baicalein).

A further object of the invention is the composition containing the co-micronized PEA-Baicalein for use in treating Benign Prostatic Hyperplasia, i.e., the use of the composition containing the co-micronized PEA-Baicalein for the preparation of a medicament for treating Benign Prostatic Hyperplasia.

The mixture of palmitoyl-ethanolamide and Baicalein in the co-micronized form will be indifferently named "co-micronized PEA-Baicalein".

These and further objects, as outlined in the appended claims, will be described in the following description. The text of the claims should be considered included in the description in order to assess the description sufficiency.

Further features and advantages of the invention will become apparent from the following description of preferred embodiments, given by way of non-limiting examples.

### Brief description of the drawings

Figure 1 depicts the granulometric distribution graph of a co-micronized PEA-Baicalein in a 9:1 ratio;
Figures 2 A-D: Serum levels of DHT (A); WB and respective densitometric analysis of 5α-red 2 (B), AR (C) and PSA (D). The values are reported as average + SEM of 6 animals for each group. ***p < 0.001 vs sham + vehicle; ## p < 0.01 vs BPH + vehicle; ### p < 0.001 vs BPH + vehicle;
Figures 3 A-C: Weight of the prostate (A); WB and respective densitometric analysis of the anti-apoptotic factor Bcl-2 (B) and the pro-apoptotic factor Bax (C). Values are reported as average + SEM of 6 animals for each group. *p < 0.05 vs sham + vehicle; **p < 0.01 vs sham + vehicle; ***p < 0.001 vs sham + vehicle; # p < 0.05 vs BPH + vehicle; ## p < 0.01 vs BPH + vehicle; ### p < 0.001 vs BPH + vehicle.

### Detailed description of the invention

The present invention relates in a first aspect to a composition comprising a mixture of palmitoyl-ethanolamide (PEA) and Baicalein in the co-micronized form. Such a mixture will henceforth also be called "co-micronized PEA-Baicalein".

Baicalein (5,6,7-trihydroxyflavone) is a flavone originally isolated from the roots of *Scutellaria baicalensis* and *Scutellaria lateriflora.* It has also been found in *Oroxylum indicum* (Indian trumpet tree) and *Thyme.* The structural formula thereof is as follows:

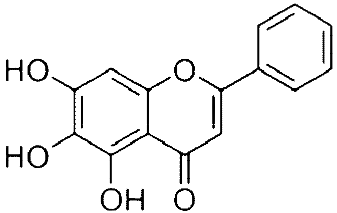

The general term "compounds in co-micronized form" refers to compounds obtained by means of a joint micronization process (i.e., simultaneous micronization of the mixture of said compounds) and having a particle size distribution, defined as a percentage by volume and measured by the laser light scattering method, represented by a distribution curve having the mode below 10 microns but above 0.5 microns.

In an embodiment, the PEA + Baicalein mixture in the co-micronized form has a particle size distribution as defined above, measured with a Malvern Mastersizer 3000 instrument with Fraunhofer calculation algorithm (1,330 dispersant refractive index; analysis model: General Purpose; laser obscuration 14.26%), in which at least 90% by volume of particles have a particle size smaller than 10 microns, preferably smaller than 9 microns.

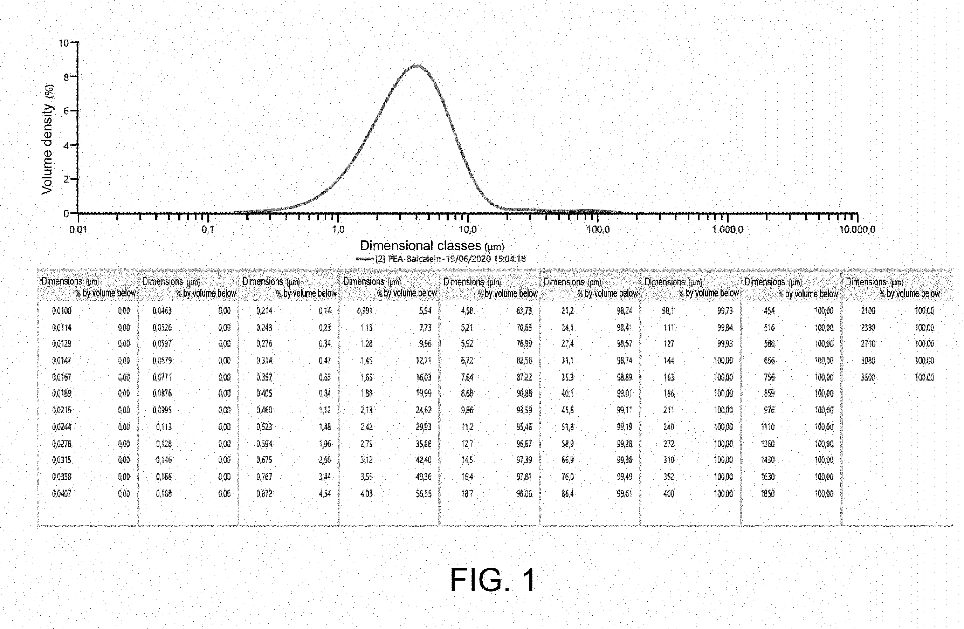

In a preferred embodiment, the PEA + Baicalein mixture in the co-micronized form has a particle size distribution as defined above, measured with a Malvern Mastersizer 3000 instrument with Fraunhofer calculation algorithm (1,330 dispersant refractive index; analysis model: General Purpose; laser obscuration 14.26%), having a mode between 3 and 4 microns and having at least 93% by volume of particles smaller than 10 microns and preferably at least 50% by volume of particles smaller than 4 microns, more preferably smaller than 3.7 microns. An example of such a particle size distribution (PEA-Baicalein ratio 9:1) is shown in fig. 1.

The micronization can be carried out in a fluid jet system (for example, Jetmill^{®} model system) which operates with spiral technology with a compressed air or nitrogen jet capable of exploiting kinetic energy - instead of mechanical energy - to crush the particles. Such apparatuses are conventional and will therefore not be further described.

The mixture of PEA and Baicalein in the co-micronized form comprises PEA and Baicalein in a PEA/Baicalein weight ratio between 10:1 and 1:1.

According to a different aspect of the invention, the composition of the invention further comprises a resveratrol glucoside, in particular trans-polydatin.

When present, the weight percentage of resveratrol glucoside, in particular trans-polydatin, with respect to the weight of the co-micronized PEA-Baicalein, is between 5% and 15%.

The composition of the present invention is usable for treating benign prostatic hyperplasia.

Therefore, it is a further object of the invention a composition comprising a mixture of PEA and Baicalein in the co-micronized form (co-micronized PEA-Baicalein), for use in the treatment of benign prostatic hyperplasia, in humans and animals.

The composition of the invention can be included in pharmaceutical or veterinary formulations and can be formulated in dosage forms for oral, buccal, parenteral, rectal, or transdermal administration.

For oral administration, the pharmaceutical compositions can be found, for example, in the form of tablets or capsules, hard or soft, prepared in the conventional fashion with pharmaceutically acceptable excipients such as binding agents (e.g., pregelatinized cornstarch, polyvinylpyrrolidone or methylcellulose hydroxypropyl); filling agents (e.g., lactose, microcrystalline cellulose or calcium hydrogen phosphate); lubricants (e.g., magnesium stearate, talc or silica); disintegrants (e.g., potato starch or sodium starch glycolate); or inhibiting agents (e.g. sodium lauryl sulfate). The tablets can be coated through methods well known in the art. The liquid preparations for oral administration can be, for example, in the form of solutions, syrups or suspensions or they can be freeze-dried products to be reconstituted, before use, with water or other suitable vehicles. Such liquid preparations can be prepared through conventional methods with pharmaceutically acceptable additives such as suspending agents (e.g. sorbitol syrup, cellulose derivatives or edible hydrogenated fats); emulsifying agents (e.g. lecithin or acacia); non-aqueous vehicles (e.g. almond oil, oily esters, ethyl alcohol or fractionated vegetable oils); and preservatives (e.g. methyl- or propyl-p-hydroxybenzoates or sorbic acid). The preparation can also conveniently contain flavorings, dyes, and sweetening agents.

The preparations for oral administration can be formulated appropriately to allow the controlled release of the active constituent.

For buccal administration, the compositions can be in the form of tablets or pills formulated in the conventional manner, adapted to an absorption at the level of the buccal mucosa. Typical buccal formulations are tablets for sub-lingual administration.

The composition of the invention can be formulated for parenteral administration by injection. The injection formulations can be presented as a single dose, for example in vials, with an added preservative. The compositions can appear in this form as suspensions, solutions, or emulsions in oily or aqueous vehicles and can contain agents of the formulation such as suspension, stabilizing and/or dispersing agents. Alternatively, the active constituent can be found in the form of a powder to be reconstituted, before use, with a suitable vehicle, for example with sterile water.

The composition of the invention can also be formulated according to rectal formulations such as suppositories or retention enemas, for example containing the basic components of the common suppositories such as cocoa butter or other glycerides.

In addition to the compositions described above, the composition of the invention can also be formulated as a deposit preparation. Such long-acting formulations can be administered by implantation (e.g., subcutaneously, transcutaneously or intramuscularly) or by intramuscular injection. Thus, for example, the composition can be formulated with appropriate polymeric or hydrophobic materials (for example in the form of an emulsion in a suitable oil) or ion exchange resins or as minimally soluble derivatives.

According to the present invention the dose of co-micronized PEA-Baicalein proposed for administration to a human (with a body weight of about 70 Kg) is 100 mg to 1000 mg or 200 mg to 800 mg of co-micronized PEA-Baicalein per dose unit. The dose unit can be administered, for example, 1 to 4 times a day. The dose will depend on the route chosen for administration. It should be considered that it may be necessary to continuously vary the dosage depending on the age and weight of the patient and also on the severity of the clinical condition to be treated. The exact dose and route of administration will ultimately be at the discretion of the attending physician or veterinarian.

A further object of the invention are also dietary compositions, food supplements and foods for special medical purposes (FSMP) comprising the co-micronized PEA-Baicalein according to the invention, possibly in the form of the composition with trans-polydatin as previously described.

"Foods for special medical purposes" mean products authorized according to the European Commission Directive to Member States no. 1999/21/EC and following. Such a term refers to a product **"intended to meet particular nutritional needs of people affected by a specific disease, disorder or medical condition"** in order to cure or help cure the specific medical condition, thus assimilating this FSMP product to a drug.

The formulations according to the invention can be prepared according to conventional methods, such as those described in Remington's Pharmaceutical Sciences Handbook, Mack Pub. Co., N.Y., USA, 17th edition, 1985 or in Remington, The Science and Practice of Pharmacy, Edited by Allen, Loyd V., Jr, 22nd edition, 2012.

### EXPERIMENTAL SECTION

### Micronization procedure

The PEA-Baicalein mixture was co-micronized in a fluid jet system (in particular, the Jetmill^{®} model system) which operates with compressed air jet "spiral technology".

Optimal micronization conditions:
- internal diameter of the micronization chamber 300 mm;
- fluid jet pressure 8 bar;
- product supply 20-25 kg/h.

### Determination of the particle size distribution

The determination of the particle size distribution was carried out on a wet sample, after 1-minute sonication.

A Malvern Mastersizer 3000 instrument operating with the LALLS (Low Angle Laser Light Scattering) technique and a Fraunhofer calculation algorithm was used (1,330 dispersant refractive index; analysis model: General Purpose; laser obscuration 14.26%).

The granulometric distribution graph for a co-micronized PEA-Baicalein in the ratio PEA/Baicalein 9:1 is shown in figure 1.

### Biological experimentation

Male Sprangue Dawley rats (200-250 gr, Envigo, Italy) fed *ad libitum* and housed in cages with controlled sleep/wake cycle were used for the *in vivo* experimentation. Before the start of the experimentation, the animals were subjected to an acclimatization period of one week considering all the experimental procedures and protocols, compliant with the principles of care and welfare of laboratory animals approved by the Italian Ministry of Health and European directives (Italian Legislative Decree 2014/26 and EU Directive 2010/63) and by the OPBA of the University of Messina.

The animals were chemically treated with testosterone propionate subcutaneously (s.c.) at a dose of 3 mg/kg dissolved in corn oil (100 µl) for 14 days to induce BPH (Benign Prostatic Hyperplasia) and randomized into 5 groups of 6 rats each treated *per os,* daily for 14 days, with 2% carboxymethylcellulose (CMC), vehicle used to suspend the Baicalein molecules, micronized PEA (in particular, in ultra-micronized form as defined in EP 2 475 352 A1) and co-micronized PEA-Baicalein:
Group 1 (Sham): healthy rats not administered testosterone propionate s.c. and treated with 2% CMC, Baicalein, ultra-micronized PEA and co-micronized PEA-Baicalein (1, 9 and 10 mg/kg, respectively).
Since no significant change was detected in the Sham groups, only the Sham + vehicle group was reported in the analyses (Sham + vehicle);
Group 2: rats administered testosterone propionate s.c. and treated with 2% CMC (BPH + vehicle);
Group 3: rats administered testosterone propionate s.c. and treated with Baicalein (1 mg/kg) suspended in 2% CMC (BPH + Baic);
Group 4: rats administered testosterone propionate s.c. and treated with ultra-micronized PEA (9 mg/kg) suspended in 2% CMC (BPH + um-PEA);
Group 5: rats administered testosterone propionate s.c. and treated with co-micronized PEA-Baicalein (10 mg/kg, in a 9:1 ratio) suspended in 2% CMC (BPH + comicro PEA-Baic).

The doses and route of administration were chosen based on previous studies (D 'Amico R et al. Effects of a new compound containing Palmitoylethanolamide and Baicalein in myocardial ischaemia/reperfusion injury in vivo, Phytomedicine, 2019. 54: 27-42).

The animals were euthanized 14 days after the start of the experimentation. Plasma and prostate were collected for DHT assay and for the identification of specific proteins, respectively. Specifically, the DHT levels were analyzed in the plasma samples with the commercially available Enzyme-linked Immunosorbent Assay (ELISA) (My BioSource). The samples were dosed with the microplate reader and the absorbance was read spectrophotometrically at 450 nm. For the protein analyses with Western Blotting (WB), the following primary antibodies were used: anti-5α-red 2 (1:500, Santa Cruz Biotechnology), anti-AR (1:500, Santa Cruz Biotechnology), anti-PSA (1:500, Santa Cruz Biotechnology), anti-Bax (1:1000, Santa Cruz Biotechnology), anti-Bcl-2 (1:1000, Santa Cruz Biotechnology) and anti-β-actin (1:5000, Santa Cruz Biotechnology). The protein expression was quantified by densitometry (BIORAD ChemiDocTM XRS+software) and normalized to the housekeeping gene β-actin.

### Results

### The co-micronized PEA-Baicalein significantly reduces serum DHT levels and the protein expression of 5α-red 2, AR and PSA responsible for BPH (Benign Prostatic Hyperplasia)

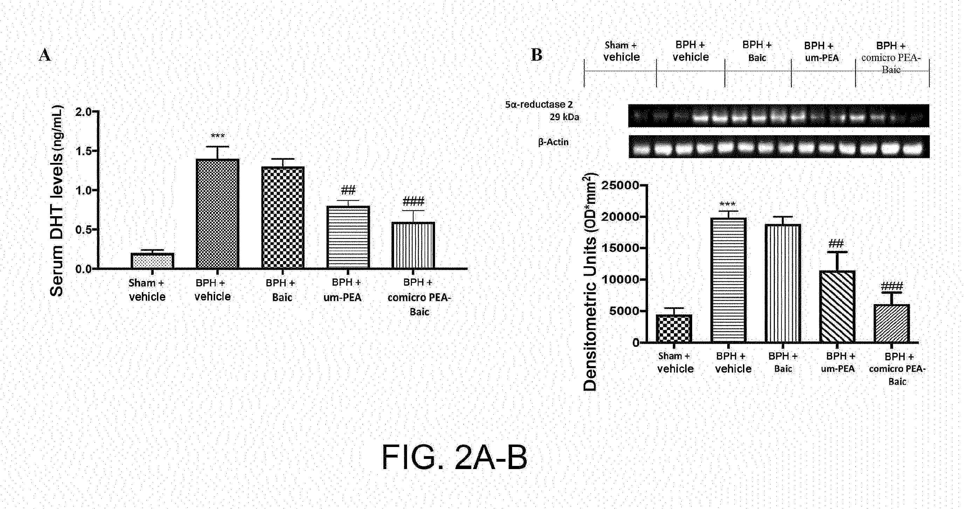

Testosterone is converted to DHT by the enzyme 5α-red 2: DHT levels were markedly elevated in the vehicle-treated BPH rat group and Baicalein alone-treated BPH animals (1 mg/kg). The daily administration of the co-micronized PEA-Baicalein (10 mg/kg in a 9:1 ratio), more than the um-PEA (9 mg/kg), significantly reduces serum DHT with respect to animals with BPH and treated with the vehicle alone (Fig. 2 A). To explain the reduction in serum DHT, the protein levels of the enzyme 5α-red 2 were analyzed. In the prostate tissue of animals with BPH treated with only vehicle, the expression of the 5α-red 2 protein increases significantly with respect to the Sham group. The treatment *per os* with co-micronized PEA-Baicalein at 10 mg/kg, more than the um-PEA alone at 9 mg/kg, significantly reduces the expression of the enzyme 5α-red 2, thus explaining the significant reduction in serum DHT. The treatment with Baicalein 1 mg/kg is not capable of reducing the expression of such an enzyme (Fig. 2 B).

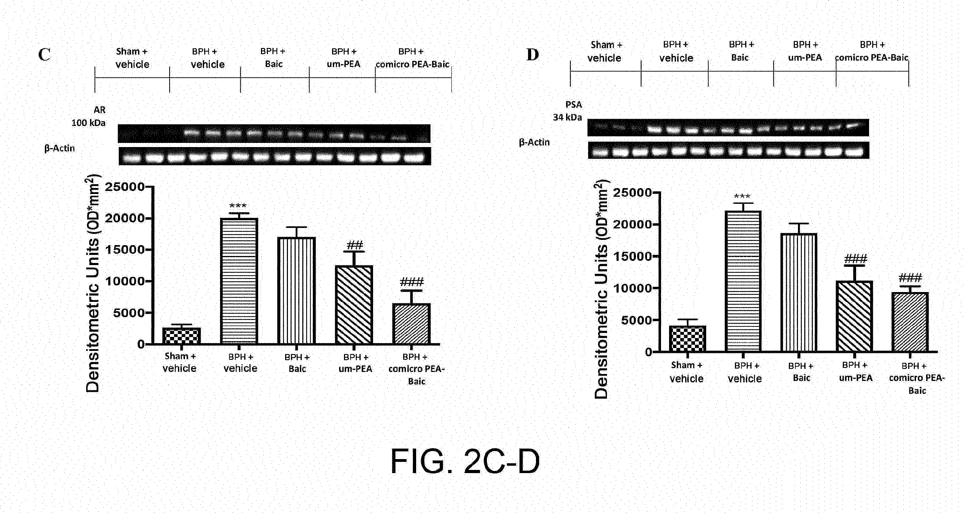

The expression of AR and PSA (respectively Fig. 2 C and 2 D) is significantly elevated in the animals with BPH with respect to the Sham group of animals. Daily treatment with co-micronized PEA-Baicalein showed a significantly greater and clearer effect, with respect to um-PEA alone, in reducing tissue levels of RA and PSA. In contrast, Baicalein at 1 mg/kg did not bring significant changes with respect to the group with BPH and treated with vehicle alone.

### Co-micronized PEA-Baicalein significantly reduces cell growth and acts on the apoptotic pathway

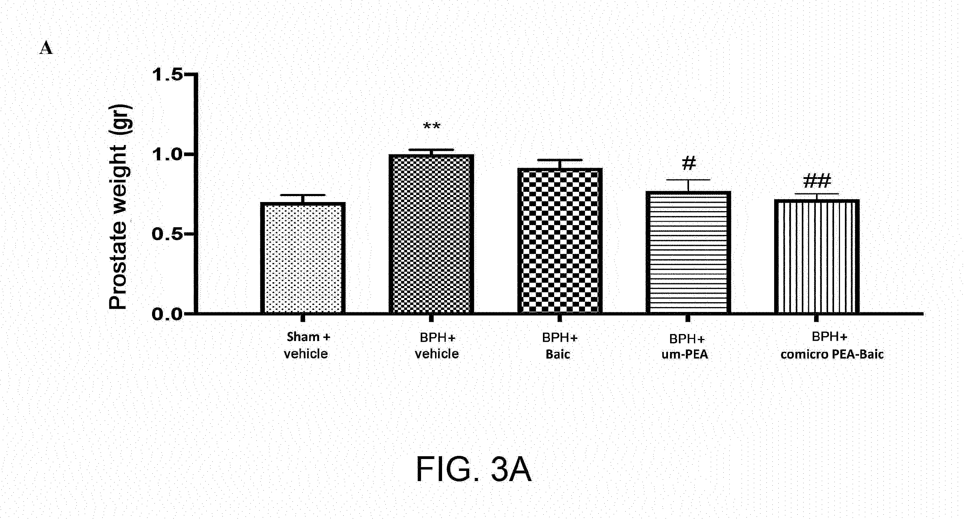

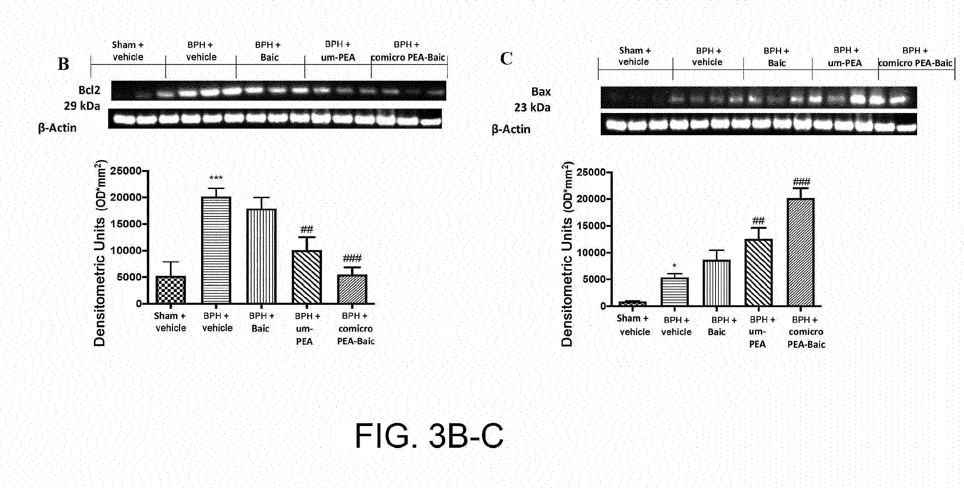

The treatment of rats with BPH with um-PEA (9 mg/kg) and with the co-micronized PEA-Baicalein (10 mg/kg), induces a progressive decrease in prostate weight (Fig. 3 A). However, the rats with BPH treated with the co-micronized PEA-Baicalein exhibit a markedly greater reduction with statistical significance with respect to the rats with BPH treated with um-PEA. Analyzing the apoptotic pathway, it is apparent how the expression of the anti-apoptotic protein Bcl-2 (Fig. 3 B) and the pro-apoptotic protein Bax (Fig. 3 C) significantly increases in the rats with BPH and treated with vehicle alone with respect to the control group (Sham). However, the increase of the anti-apoptotic protein Bcl-2 has a much greater increase than that of the pro-apoptotic protein Bax, which favors cell proliferation.

The synergistic action of the co-micronized PEA-Baicalein at the dose of 10 mg/kg significantly reduces the levels of the anti-apoptotic factor Bcl-2, significantly increasing the expression of the pro-apoptotic factor Bax with respect to the group with BPH treated with the vehicle alone. A decidedly minor effect and with lower statistical significance was observed following the administration of um-PEA alone. In contrast, the treatment with Baicalein 1 mg/kg did not show a statistically significant change with respect to the group of animals with BPH and treated with vehicle alone.

From the above, it is apparent that the co-micronized PEA-Baicalein shows a significant synergistic effect in the treatment of Benign Prostatic Hyperplasia, an entirely unexpected effect in light of what is known in the literature and the data related to the individual substances (PEA and Baicalein) reported above.

The invention will now be further described by means of the following formulation examples.

### Formulation examples

### Example 1

Each tablet contains:

| | |
|---|---|
| - co-micronized PEA-Baicalein (9:1) | 300.00 mg |
| - Microcrystalline cellulose | 78.47 mg |
| - Sodium croscarmellose | 45.00 mg |
| - Polyvinylpyrrolidone | 10.00 mg |
| - Magnesium stearate | 4.00 mg |
| - Polysorbate 80 | 2.00 mg |

### Example 2

Each tablet contains:

| | |
|---|---|
| - co-micronized PEA-Baicalein (9:1) | 600.00 mg |
| - Microcrystalline cellulose | 156.94 mg |
| - Sodium croscarmellose | 90.00 mg |
| - Polyvinylpyrrolidone | 20.00 mg |
| - Magnesium stearate | 8.00 mg |
| - Polysorbate 80 | 4.00 mg |

### Example 3

Each two-layer tablet contains:
Layer a

| | |
|---|---|
| - co-micronized PEA-Baicalein (8:2) | 400.00 mg |
| - Pharmacologically acceptable excipients | 200.00 mg |

Layer b

| | |
|---|---|
| - Trans-polydatin | 40.00 mg |
| - Pharmacologically acceptable excipients | 25.00 mg |

### Example 4

Each suppository contains:

| | |
|---|---|
| - co-micronized PEA-Baicalein (9:1) | 400.00 mg |
| - Lipophilic mass as needed to | 2.00 g |

### Example 5

Each suppository contains:

| | |
|---|---|
| - co-micronized PEA-Baicalein (9:1) | 500.00 mg |
| - Trans-polydatin | 40.00 mg |
| - Water soluble mass (PEG 400 40% + PEG 4000 60%) as needed to | 2.50 g. |

## Claims

1. A composition comprising a mixture of palmitoyl-ethanolamide (PEA) and Baicalein in the co-micronized form.

2. The composition according to claim 1, wherein the mixture of palmitoyl-ethanolamide (PEA) and Baicalein in the co-micronized form has a particle size distribution, measured by a Malvern Mastersizer 3000 instrument with Fraunhofer calculation algorithm, where at least 90% by volume of particles has a particle size of less than 10 microns or less than 9 microns.

3. The composition according to claim 1 or 2, wherein the mixture of palmitoyl-ethanolamide (PEA) and Baicalein in the co-micronized form has a particle size distribution, measured by a Malvern Mastersizer 3000 instrument with Fraunhofer calculation algorithm, having a mode between 3 and 4 microns and having at least 93% by volume of particles smaller than 10 microns and preferably at least 50% by volume of particles smaller than 4 microns or smaller than 3.7 microns.

4. The composition according to any one of claims 1 to 3, wherein the mixture of PEA and Baicalein in the co-micronized form comprises PEA and Baicalein in a PEA/Baicalein weight ratio between 10:1 and 1:1.

5. The composition according to any one of claims 1 to 4, wherein the composition further comprises a resveratrol glucoside, preferably trans-polydatin.

6. The composition according to claim 5, wherein the weight percentage of the resveratrol glucoside, with respect to the weight of the co-micronized PEA-Baicalein, is between 5% and 15%.

7. A composition according to any one of claims 1 to 6, for use in the treatment of Benign Prostatic Hyperplasia in humans and animals.

8. The composition for use according to claim 7, wherein said PEA and said Baicalein, in said co-micronized PEA-Baicalein, explicate a synergistic effect.

9. The composition for use according to claim 7 or 8, wherein said co-micronized PEA-Baicalein decreases the expression of the anti-apoptotic protein Bcl-2 and increases the expression of the pro-apoptotic protein Bax in prostate tissue.

10. A human or veterinary pharmaceutical formulation, comprising the composition according to any one of claims 1 to 6.

11. The formulation according to claim 10, formulated in dosage forms for oral, buccal, parenteral, rectal or transdermal administration.

12. The formulation according to claim 10 or 11, wherein the co-micronized PEA-Baicalein is contained in amounts between 100 mg and 1000 mg per dose unit.

13. A dietary composition, food supplement or food for special medical purposes (FSMP), or feed, or nutritional supplements for animals, comprising the composition according to any one of claims 1 to 6.

14. A human or veterinary pharmaceutical formulation according to any one of claims 10 to 12, for use in the treatment of Benign Prostatic Hyperplasia.
